Europäisches Patentamt

(19) European Patent Office

Office européen des brevets

(11) Publication number : **0 370 632 B1**

(12) **EUROPEAN PATENT SPECIFICATION**

(45) Date of publication of patent specification :
29.07.92 Bulletin 92/31

(51) Int. Cl.⁵ : **A61K 31/35,** A61K 9/72,
// (A61K31/35, 31:135)

(21) Application number : 89311021.3

(22) Date of filing : 25.10.89

(54) **Pharmaceutical solution containing salbutamol and cromoglycic acid.**

(30) Priority : **04.11.88 GB 8825892**

(43) Date of publication of application :
**30.05.90 Bulletin 90/22**

(45) Publication of the grant of the patent :
**29.07.92 Bulletin 92/31**

(84) Designated Contracting States :
**AT DE NL SE**

(56) References cited :
**US-A- 3 957 965
CHEMICAL ABSTRACTS, vol. 108, no. 12, 21st
March 1988; IACONO, Marc: "An investigation
of the compatibility of ipratropiumand sodium
cromoglycate nebulizer solutions", page 440,
abstract no. 101235j, & AUST. J. HOSP.
PHARM. 1987, 17(3), 158-61**

(73) Proprietor : **FISONS plc
Fison House Princes Street
Ipswich Suffolk IP1 1QH (GB)**

(72) Inventor : **Chawla, Brindra Paul Singh
45 Boundary Road
West Bridgford Nottingham (GB)**
Inventor : **Steele, Gerald
37 Castledine Street Extension
Loughborough Leicestershire (GB)**

(74) Representative : **Wright, Robert Gordon McRae
FISONS plc 12 Derby Road
Loughborough Leicestershire LE11 0BB (GB)**

EP 0 370 632 B1

## Description

This invention relates to pharmaceutical solutions, methods for their preparation and methods of treatment involving their use.

Sodium cromoglycate, the disodium salt of 1,3-bis(2-carboxy-chromon-5-yloxy)-propan-2-ol (cromoglycic acid), has been known for many years for the prophylactic treatment of bronchial asthma by inhalation to the lung. The drug is most commonly administered as a powder but also as a nebulised aqueous solution.

(2-Tert-butylamino)-1-(4-hydroxy-3-hydroxymethyl phenyl)ethanol, which is known generically as salbutamol, is known to possess bronchodilatory properties and is also used in the treatment of bronchial asthma. It too is commonly administered as an aerosol or by nebulisation of an aqueous solution.

The possible therapeutic value of a solution of sodium cromoglycate and salbutamol has been recognised for some time. However, solutions containing both active ingredients have been found to be unstable. To overcome this problem, the two compounds have either been administered separately or else solutions containing both active ingredients have been made up immediately prior to use by adding one of the active ingredients to a solution of the other. For example, the contents of commercially available ampoules containing solutions of the two compounds have been mixed. In other cases, sodium cromoglycate powder has been dissolved in a salbutamol solution immediately prior to administration. Iacono et al, Aust. J. Hosp. Pharm., 1987, 17, 158 (Chemical Abstracts, 1988, Vol. 108:101235j) disclose compositions comprising sodium cromoglycate, salbutamol sulphate and ipratropium bromide. However, no suggestion is made of the criticality of pH to the stability of such compositions.

We have now surprisingly found that the stability of solutions containing sodium cromoglycate and salbutamol can be substantially increased by adjusting the pH of the solution to a relatively narrow range.

Thus, according to the invention there is provided an aqueous solution containing cromoglycic acid, or a pharmaceutically acceptable salt thereof, as Active Ingredient A, salbutamol, or a pharmaceutically acceptable salt thereof, as Active Ingredient B, and an acid pH modifying agent, characterised in that the solution has a pH of greater than 3.8 and less than 4.8, provided that the solution does not contain ipratropium bromide.

The solution according to the invention is advantageous in that it shows a markedly reduced tendency to deteriorate on storage than do known solutions of the Active Ingredients and hence can be stored for considerably longer times. This eliminates the need for the solution to be made up immediately prior to use and enables the solution to be supplied in ready-to-use form, thereby greatly increasing the convenience to the user.

Active Ingredient A is preferably in the form of the disodium salt, sodium cromoglycate.

Active Ingredient B is preferably in the form of salbutamol sulphate.

The solution preferably has a pH greater than 4.0. The pH of the solution is preferably less than 4.75, more preferably less than 4.5. It is particularly preferred that the solution has a pH of between 4.0 and 4.5.

The pH is adjusted by the incorporation of a suitable acid pH-modifying agent ie an ingredient which has a substantial effect on the pH of the solution. Any suitable pharmaceutically acceptable acid may be used; such acids include organic acids, eg acetic acid, and, more preferably, mineral acids, eg hydrochloric acid and sulphuric acid. It is particularly preferred that the pH-modifying agent be chosen such that it does not introduce any additional ionic species into the solution. For example, when Active Ingredient B is present as salbutamol sulphate, as is preferred, then a favoured pH-modifying agent is sulphuric acid. The quantity of pH-modifying agent required will of course be such as to confer on the solution the desired pH.

The solution preferably contains from about 0.1 to 10% w/v of Active Ingredient A, more preferably 0.1 to 5%, and especially less than about 2.5%, eg 1% w/v.

The solution preferably contains from about 0.01 to 1.0 % w/v of Active Ingredient B, more preferably 0.01 to 0.5%, and especially less than 0.25%, eg 0.1% or 0.05% w/v.

Apart from Active Ingredient A, Active Ingredient B and the pH-modifying agent, the solution preferably contains no further ingredients. However, other excipients may, if desired, be included.

For example, the solution may contain one or more buffering agents. Buffering agents which may be used will be readily apparent to those skilled in the art but, by way of example, the following may be mentioned:

sodium dihydrogen orthophosphate (sodium acid phosphate BP), di-sodium hydrogen phosphate (sodium phosphate BP), sodium citrate/citric acid, and boric acid/sodium borate.

Other excipients which may be present in the solution include chelating or sequestering agents. Suitable chelating or sequestering agents include sodium carboxymethyl cellulose, citric, tartaric and phosphoric acids, and amino carboxylate compounds. The preferred chelating agent, however, is ethylenediamine tetraacetic acid or a salt thereof, especially the disodium salt.

The solution may also be made isotonic with physiological fluids by the incorporation of a suitable tonicity agent. Preferred tonicity-adjusting agents are non-ionic species, including polyols such as glycerol, polypropylene glycol, polyethylene glycol, sorbitol, mannitol, and sugars, eg dextrose, fructose, lactose, mannose and sucrose. Particularly preferred tonicity-adjusting agent are the sugars and, more preferably, mannitol.

The concentration of tonicity-adjusting agent in the solution will vary, depending of course on the particular tonicity-adjusting agent used. However, in general the concentration of tonicity-adjusting agent should be such as to make the solution isotonic with body fluids, ie to confer on the solution a tonicity of about 270 to 330 mOsm.

The proportion and concentration of buffers, if included, and other excipients may be varied within fairly wide ranges, provided the resulting solution is stable and non-irritant when applied to the appropriate tissues. The maximum total concentration of excipients and buffers is preferably less than 10% w/v.

The solution may be made up by dissolving the Active Ingredients and excipients (if used) in freshly distilled water and adjusting the pH. The solution is preferably made up at a temperature of between 10°C and 50°C.

The solution may be put up in unit dosage form, in which case preservatives may be incorporated but are generally not necessary. Alternatively the solution may be put up in multi-dose form. In general it will be necessary to incorporate one or more preservatives into multi-dose solutions to ensure that the solution remains sterile after initial use. The preservatives which may be used are those which are conventionally employed in pharmaceutical solutions and the concentration to be used should be such as to ensure effective preservation, ie such that bacterial growth is inhibited.

Unit doses of the solution are preferably packed in glass or plastics ampoules containing from about 1 to 5ml of solution, eg 2ml. The solution is preferably sterilised; where the solution is packed in glass ampoules sterilisation is preferably by autoclaving, where the solution is packed in plastics ampoules sterilisation is preferably carried out by aseptic filling through a sterile filter.

Each dose preferably comprises from about 1 to 100mg, more preferably from 1 to 50mg, of Active Ingredient A and from about 0.05 to 10mg, more preferably 0.05 to 5mg, of Active Ingredient B.

It is preferred that the dose of salbutamol administered be such as to give a sustained rather than a transitory action.

Multi-dose solutions may be packaged in volumes of 5 to 300ml, eg 60, 120 and 240 ml. We prefer multi-dose solutions to be packaged such that unit volumes of the solution may be accurately dispensed.

The solution of the invention is useful in the treatment of the conditions generically known as reversible obstructive airways disease. Such conditions include allergic asthma, intrinsic asthma, bronchitis and the nasal and bronchial obstructions associated with the common cold. The solution is generally administered by nebulisation.

The dosage to be administered will of course vary with the precise nature of the condition to be treated and with its severity. However, in general a total daily dosage of from about 10 to 100mg Active Ingredient A and 1 to 10mg Active Ingredient B is found to be satisfactory. The daily dosage may be administered in divided doses, eg from 2 to 4 times a day. More frequent dosage may of course be used if required.

The invention is illustrated, but in no way limited, by the following Examples.

Example 1

Non-preserved nebuliser solution

Ingredients

| | |
|---|---|
| Sodium cromoglycate | 1.0 % w/v |
| Salbutamol sulphate | 0.1 % |
| Sulphuric acid | q.s. |
| Purified water | to 100% |

Method

The sodium cromoglycate (10g) and salbutamol sulphate (1g) were dissolved in purified water (900ml). The pH of the solution was adjusted to between 4.0 and 4.5 by addition of sulphuric acid. The volume was made up to 1000ml with purified water.

The solution was sterile-filled into plastics ampoules which were then sealed.

Example 2

Non-preserved isotonic nebuliser solution

Ingredients

| | |
|---|---|
| Sodium cromoglycate | 1.0% w/v |
| Salbutamol sulphate | 0.1% |
| Mannitol | q.s. |
| Sulphuric acid | q.s. |
| Purified water | to 100% |

Method

The sodium cromoglycate (10g), salbutamol sulphate (1g) and mannitol (sufficient to give a tonicity in the final solution of 290mOsm) were dissolved in purified water (900ml). The pH was adjusted to between 4.0 and 4.5 by the addition of sulphuric acid and the volume made up to 1000ml with purified water. The solution was filled into glass ampoules (2ml) which were then sealed and sterilised by autoclaving.

**Claims**

1. An aqueous solution containing cromoglycic acid, or a pharmaceutically acceptable salt thereof, as Active Ingredient A, salbutamol, or a pharmaceutically acceptable salt thereof, as Active Ingredient B, and an acid pH-modifying agent, characterised in that the solution has a pH of greater than 3.8 and less than 4.8, provided that the solution does not contain ipratropium bromide.

2. A solution according to Claim 1, wherein Active Ingredient A is sodium cromoglycate.

3. A solution according to Claim 1 or Claim 2, wherein Active Ingredient B is salbutamol sulphate.

4. A solution according to any one of the preceding claims, wherein the pH is greater than 4.0.

5. A solution according to any one of the preceding claims, wherein the pH is less than 4.5.

6. A solution according to any one of the preceding claims, wherein the pH is between 4.0 and 4.5.

7. A solution according to any one of the preceding claims, which contains salbutamol sulphate and sulphuric acid.

8. A solution according to any one of the preceding claims, which contains from 0.1 to 5% w/v of Active Ingredient A.

9. A solution according to any one of the preceding claims, which contains from 0.01 to 0.5% w/v of Active Ingredient B.

**Patentansprüche**

1. Wässerige Lösung, die Cromoglicinsäure oder ein pharmazeutisch annehmbares Salz hievon als aktiven Bestandteil A, Salbutamol oder ein pharmazeutisch annehmbares Salz hievon als Bestandteil B und ein saures pH-Modifiziermittel enthält, dadurch gekennzeichnet, daß sie einen pH von höher als 3,8 und weniger als 4,8 aufweist, mit der Maßgabe, daß die Lösung Ipratropiumbromid nicht enthält.

2. Lösung nach Anspruch 1, in der der aktive Bestandteil A Natriumcromoglicat ist.

3. Lösung nach Anspruch 1 oder 2, in der der aktive Bestandteil B Salbutamolsulfat ist.

4. Lösung nach einem der vorhergehenden Ansprüche, in der der pH höher als 4,0 ist.

5. Lösung nach einem der vorhergehenden Ansprüche, in der der pH niedriger als 4,5 ist.

6. Lösung nach einem der vorhergehenden Ansprüche, in der der pH 4,0 bis 4,5 beträgt.

7. Lösung nach einem der vorhergehenden Ansprüche, die Salbutamolsulfat und Schwefelsäure enthält.

8. Lösung nach einem der vorhergehenden Ansprüche, die 0,1 bis 5 % w/v des aktiven Bestandteiles A enthält.

9. Lösung nach einem der vorhergehenden Ansprüche, die 0,01 bis 0,5 % w/v des aktiven Bestandteiles B enthält.

**Revendications**

1. Solution aqueuse contenant de l'acide cromoglycique ou un sel pharmaceutiquement acceptable de celui-ci comme Constituant Actif A, du salbutamol ou un sel pharmaceutiquement acceptable de celui-ci comme Constituant Actif B, et un agent acide modifiant le pH, caractérisée en ce que la solution a un pH supérieur à 3,8 et inférieur à 4,8, avec la restriction que la solution ne contient pas de bromure d'ipatropium.

2. Solution suivant la revendication 1, dans laquelle le Constituant Actif A est le cromoglycate sodique.

3. Solution suivant la revendication 1 ou 2, dans laquelle le Constituant Actif B est le sulfate de salbutamol.

4. Solution suivant l'une quelconque des revendications précédentes, dans laquelle le pH est supérieur à 4,0.

5. Solution suivant l'une quelconque des revendications précédentes, dans laquelle le pH est inférieur à 4,5.

6. Solution suivant l'une quelconque des revendications précédentes, dans laquelle le pH se situe entre 4,0 et 4,5.

7. Solution suivant l'une quelconque des revendications précédentes, qui contient du sulfate de salbutamol et de l'acide sulfurique.

8. Solution suivant l'une quelconque des revendications précédentes, qui contient 0,1 à 5% w/v du Constituant Actif A.

9. Solution suivant l'une quelconque des revendications précédentes, qui contient 0,01 à 0,5% w/v du Constituant Actif B.